# EUROPEAN PATENT SPECIFICATION

(11) **EP 4 525 801 B1**
(45) Date of publication and mention of the grant of the patent: **01.07.2026**
(21) Application number: 23726012.0
(22) Date of filing: 09.05.2023
(51) Int. Cl.: A61F 13/476, A61F 13/514, A61F 13/58, A61F 13/62

(54) **ABSORBENT ARTICLE WITH FASTENING SYSTEM**
SAUGFÄHIGER ARTIKEL MIT BEFESTIGUNGSSYSTEM
ARTICLE ABSORBANT DOTÉ D'UN SYSTÈME DE FIXATION

(30) Priority: 17.05.2022 EP 22173834; 29.11.2022 EP 22210250; 01.12.2022 EP 22210953; 19.12.2022 EP 22214735
(43) Date of publication of application: 26.03.2025
(60) Divisional application: 26181171.5
(73) Proprietor: Ontex BV, 9255 Buggenhout (BE); Ontex Group NV, 9320 Erembodegem (BE)
(72) Inventor: REUTER, Agnes Dominika, 50823 Köln (DE); IDELSON, Alissa, 53359 Rheinbach (DE); PANNWITT, Stefanie, 56727 Mayen (DE)
(74) Representative: Macchetta, Andrea
(86) International application number: PCT/EP2023/062311
(87) International publication number: WO 2023/222463

(56) References cited:
- EP-B1- 0 974 326
- WO-A1-2020/074400
- US-A1- 2007 039 142
- US-A1- 2017 281 428

## Description

### TECHNICAL FIELD

The disclosure relates to absorbent articles such as disposable absorbent articles, preferably selected from diapers (whether for baby or adults) free of discrete/separate frontal tape/fastener and/or landing zone.

### BACKGROUND

Examples in the literature exist wherein investigations have been carried out on effective fastener solutions for diapers.

One such early examples is described in WO 03/034966 A1. What is described is a disposable absorbent article having longitudinal side edges and transverse end edges, a first end region, and a second end region opposite of the first end region. The absorbent article comprises a liquid pervious topsheet, a liquid impervious backsheet, an absorbent core disposed between the liquid pervious topsheet and the liquid impervious backsheet, and an outer nonwoven layer disposed on an outer surface of the liquid impervious backsheet. The absorbent article further comprises a closure member. The closure member is joined adjacent to the longitudinal side edge in the first end region. The closure member comprises a hook fastening material engageable with the outer nonwoven layer for forming a closure for the absorbent article. The liquid impervious backsheet comprises landing zone graphics disposed in the second end region. The landing zone graphics are covered by the outer nonwoven layer and visible through the outer nonwoven layer. Notable disadvantages however included high cost due to the necessarily higher basis weight for the outer cover layer that acted as landing zone when applied through the entire backsheet of the diaper as well as higher environmental impact due to the generally higher amount of disposable material being generated.

Some work to overcome such shortcomings has been done and is exemplified in WO2020/074400 A1. What is described therein is a disposable diaper, particularly a baby diaper, having a cover sheet close to the body, having a back sheet away from the body, and having at least one fastening flap, the back sheet having a nonwoven layer and the fastening flap having hook regions and adhesive regions, each of which come into contact with the back sheet when in the held state. According to the description, the nonwoven layer of the back sheet is bonded in a bonding pattern consisting of pattern elements and, in the held state, the adhesive regions within the pattern elements take up an average area proportion of at least 20% relative to the total area of the particular pattern element. The particular combination of a nonwoven with a bonding pattern (i.e. a nonwoven with an embossment pattern) used as an outer cover with fasteners having a combination of hooks and adhesive and where the adhesive being arranged to occupy such a defined area of the pattern elements was found to allow for improved mechanical and chemical engagement of the fasteners directly to the outer cover without the need of a dedicated landing zone being added. Described nonwovens have a basis weight of between 10 gsm and 20 gsm.

Further prior art is known from US2007/039142 and EP0974326.

Although there have already been significant advancements in this field, there still remains a need to further improve the fastening force whilst limiting the delamination effects caused by excessive bonding of the fasteners. It may further be advantageous to improve the environmental impact of absorbent articles such as diapers and yet in a cost effective manner whilst retaining high performance and good fastening of the articles in-use.

### SUMMARY

The disclosure relates to a disposable absorbent article comprising: a liquid permeable topsheet; a liquid impermeable backsheet; and an absorbent core comprising absorbent material therein and being sandwiched between said topsheet and backsheet; the topsheet, backsheet and absorbent core together forming a chassis of said article, wherein said chassis comprises a perimeter formed by first and second transverse edges and first and second longitudinal edges connecting the first and second transverse edges; a longitudinal centerline (y) extending substantially parallel to said first and second longitudinal edges and interposed therebetween such to divide said chassis into a first longitudinal half between said longitudinal centerline (y) and said first longitudinal edge and a second longitudinal half between said longitudinal centerline (y) and said second longitudinal edge; and a transverse centerline (x) extending substantially parallel to said first and second transverse edges and interposed therebetween such to divide said chassis into a first transverse half between said transverse centerline (x) and said first transverse edge and a second transverse half between said transverse centerline (x) and said second transverse edge; wherein said article further comprises an outer cover positioned on a garment-facing side of said backsheet and covering at least 75% of an area defined by said perimeter of said chassis; and a fastening system consisting of one or more male fasteners and a female fastening material; and wherein the female fastening material comprises a landing zone arranged to receive said one or more male fasteners thereon to provide a fastening of said one or more male fasteners to said female fastening material, wherein said female fastening material comprises, preferably consists of, said outer cover; wherein the one or more male fasteners comprise one or more first areas of adhesive **A1** and one or more second areas of mechanical fastening elements **A2** and wherein the total area ratio Σ**A1** / Σ**A2** is from about 0.4 to about 1 and wherein the outer cover may be an embossed nonwoven comprising high-fiber-density areas and low-fiber-density areas, wherein the low-fiber-density areas are at least substantially enclosed by high-fiber-density areas, and wherein the high-fiber-density areas are disposed in a repeated pattern corresponding to embossments of said nonwoven.

### BRIEF DESCRIPTION OF THE FIGURES

**Fig. 1** Is an illustration, bottom planar view (backsheet-side), of an absorbent article according to an embodiment herein.
**Fig. 2** Is an illustration, bottom planar view (backsheet-side), of an absorbent article according to an embodiment herein.
**Fig. 3** Is an illustration, cross-section, of an absorbent article according to an embodiment herein.
**Fig. 4** Is an illustration of a portion of a male fastener.
**Fig. 5** Is an illustration of a portion of a male fastener according to an embodiment herein.
**Fig. 6** Is an illustration of a portion of a male fastener according to an embodiment herein.
**Fig. 7** Illustrates a portion of an outer cover according to an embodiment herein.
**Fig. 8A-B** are illustrations of a male fastener according to embodiments herein.
**Fig. 9** Illustrates a schematic of an embossment pattern comprised in an outer cover.
**Fig. 10** Illustrates a schematic of an embossment pattern comprised in an outer cover.
**Fig. 11** Illustrates a fastener according to an embodiment herein.

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural referents unless the context clearly dictates otherwise. By way of example, "a compartment" refers to one or more than one compartment.

"About" or "substantially" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed disclosure. However, it is to be understood that the value to which the modifier "about" or "substantially" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The expression "% by weight" or "%wt" (weight percent), here and throughout the description unless otherwise defined, refers to the relative weight of the respective component based on the overall weight of the formulation.

The use of the term "layer" can refer, but is not limited, to any type of substrate, such as a woven web, nonwoven web, films, laminates, composites, elastomeric materials, absorbent materials (such as SAP and cellulose fibers/fluff mixtures), or the like. A layer can be liquid and air permeable, permeable to air but impermeable to liquids, impermeable both to air and liquid, or the like. When used in the singular, it can have the dual meaning of a single element or a plurality of elements, such as a laminate or stacked plural sub-layers forming a common layer.

The terms "nonwoven", "nonwoven layer" or "nonwoven web" are used interchangeably to mean an engineered fibrous assembly, primarily planar, which has been given a designed level of structural integrity by physical and/or chemical means, excluding weaving, knitting or papermaking (ISO 9092:2019 definition). The directionally or randomly orientated fibers, are bonded by friction, and/or cohesion and/or adhesion. The fibers may be of natural or synthetic origin and may be staple or continuous filaments or be formed in situ. Commercially available fibers have diameters ranging from less than about 0.001 mm to more than about 0.2 mm and they come in several different forms such as short fibers (known as staple, or chopped), continuous single fibers (filaments or monofilaments), untwisted bundles of continuous filaments (tow), and twisted bundles of continuous filaments (yam). Nonwoven webs can be formed by many processes such as meltblowing, spunbonding, solvent spinning, electrospinning, carding and airlaying. The basis weight of nonwoven webs is usually expressed in grams per square meter (g/m2 or gsm).

"Gsm" as used herein means grams per square meter, and is a measure of the basis weight, which is an industry standard term that quantifies the thickness or unit mass of a film or laminate product.

"Tear strength" or "tear force," as used herein reflects the ease or difficulty by which the film can be torn, and is expressed in units of grams. Herein, tear strength may be measured by the Elmendorf notched tear test, ASTM D-1922, incorporated herein by reference and/or by the Trapezoid tear test ("trap test"), as described herein or according to ASTM D-5587. The test may be performed with either a notched or an unnotched film and in either the cross direction (CD) or machine direction (MD) direction. Unless otherwise specified, herein tear strength is notched tear strength. It is noted that tear strength is related to film thickness, and any comparison of tear strengths should take into account the relative basis weights of the comparative samples.

"Tensile strength," means the load required to induce a break ("load at break") in the film in either the CD or the MD. Tensile strength is expressed in units of N/cm or equivalent units thereof, and is determined by ASTM method D822-02, using the following parameters: Sample Direction = MD x CD; Sample size = 1 inch width x 6 inch length; Test speed = 20 in/min; Grip distance = 2 inch. Grip size = 3 inch wide rubber faced grips evenly gripping sample.

The terms "spunlace" or "spunlace nonwoven" as used herein refer to nonwoven fabrics or materials that are made by hydroentangling webs of fibers (and/or fibers) with high energy water jets for example as basically described in Evans et al. US Patent No. 3,485,706. The webs may be made of a variety of fibers such as polyester, rayon, cellulose (cotton and wood pulp), acrylic, and other fibers as well as some blends of fibers. The fabrics may be further modified to include antistatic and antimicrobial properties, etc. by incorporation of appropriate additive materials into the fiber or fiber webs.

As used herein, the term "cellulosic" or "cellulose" is meant to include any material having cellulose as a major constituent, and specifically comprising at least 50 percent by weight cellulose or a cellulose derivative. Thus, the term includes cotton, typical wood pulps, nonwoody cellulosic fibers, cellulose acetate, cellulose triacetate, rayon, thermomechanical wood pulp, chemical wood pulp, debonded chemical wood pulp, milkweed, or bacterial cellulose.

The term "disposable" refers to absorbent articles and/or inserts that generally are not intended to be laundered or otherwise restored or reused as absorbent articles, i.e., they are intended to be discarded after a single use and, preferably, to be recycled, composted or otherwise disposed of in an environmentally compatible manner.

The term "disposed" is used to mean that an element(s) is formed (joined and positioned) in a particular place or position as a unitary structure with other elements or as a separate element joined to another element.

The terms "interior" and "exterior" refer respectively to the location of an element that is intended to be placed against or toward the body of a wearer when an absorbent article is worn and the location of an element that is intended to be placed against or toward any clothing that is worn over the absorbent article. Synonyms for "interior" and "exterior" include, respectively, "inner" and "outer", as well as "inside" and "outside", or "body-facing" and "garment-facing". Also, when the absorbent article is oriented such that its interior faces upward, e.g., when it is laid out in preparation for setting the wearer on top of it, synonyms include "upper" and "lower" and "top" and "bottom", respectively.

Terms "WVTR" or "water vapor transmission rate" are used herein interchangeably and are taken to mean a measure of film breathability. WVTR is expressed in units of g H₂O/24-hours/m² or equivalent units thereof, and may be measured according to ASTM method D-6701-01.

The term "joined" refers to configurations whereby an element is directly secured to another element by attaching the element directly to the other element, and configurations whereby an element is indirectly secured to another element by attaching the element to intermediate member(s) which in turn are attached to the other element.

The term "lateral" or "transverse" refers to a direction running at a 90 degree angle to the longitudinal direction and when combined with the term "substantially" includes directions within ±45° of the lateral direction.

The term "longitudinal" refers to a direction running parallel to the maximum linear dimension of the article and when combined with the term "substantially" includes directions within ±45° of the longitudinal direction.

"Plant-based fibers", as used herein, includes both harvested fibers and synthetic fibers that comprise bio-based content. Harvested plant-based fibers include cellulosic matter, such as wood pulp; seed hairs, such as cotton; stem (or bast) fibers, such as flax and hemp; leaf fibers, such as sisal; and husk fibers, such as coconut. Assessment of the renewably based carbon in a material can be performed through standard test methods. Using radiocarbon and isotope ratio mass spectrometry analysis, the bio-based content of materials can be determined. ASTM International has established a standard method for assessing the bio-based content of materials. The ASTM method is designated ASTM D6866-10.The application of ASTM D6866-10 to derive a bio-based content and the analysis is performed by deriving a ratio of the amount of organic radiocarbon (14C) in an unknown sample to that of a modern reference standard. The ratio is reported as a percentage with the units "pMC" (percent modern carbon). The modern reference standard used in radiocarbon dating is a NIST (National Institute of Standards and Technology) standard with a known radiocarbon content equivalent approximately to the year AD 1950. AD 1950 was chosen since it represented a time prior to 7dhesiv-nuclear weapons testing which introduced large amounts of excess radiocarbon into the atmosphere with each explosion (termed "bomb carbon"). The AD 1950 reference represents 100 pMC.

"Bio-based content" refers to the amount of carbon from a renewable resource in a material as a percent of the mass of the total organic carbon in the material, as determined by ASTM D6866- 10, method B. In order to apply the methodology of ASTM D6866-10 to determine the bio-based content of any absorbent article or component thereof, a sample can be ground into particulates less than about 20 mesh using known grinding methods (e.g., WILEY mill), and a representative sample of suitable mass taken from the randomly mixed particles. Alternatively, if the sample is merely a layer of material, then the layer itself can be analyzed without the need for a pre-grinding step. Note that any carbon from inorganic sources such as calcium carbonate is not included in determining the bio-based content of the material.

Embodiments of the articles according to the disclosure will now be described. It is understood that technical features described in one or more embodiments maybe combined with one or more other embodiments without departing from the intention of the disclosure and without generalization therefrom, especially when such combinations are explicitly or implicitly inferred.

### THE ABSORBENT ARTICLE

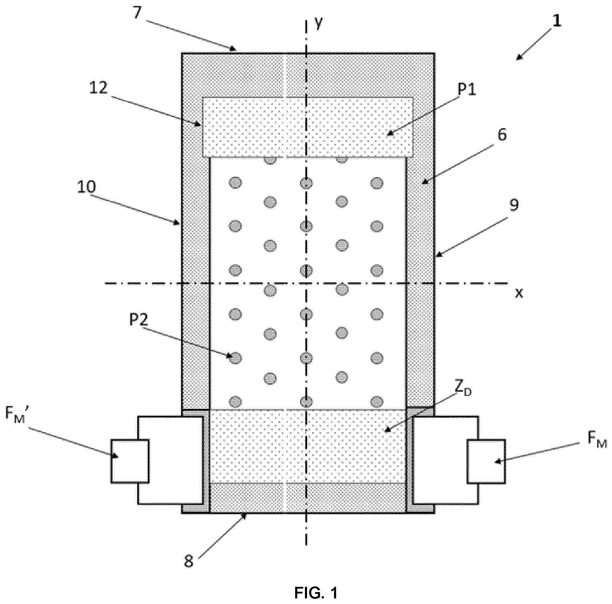

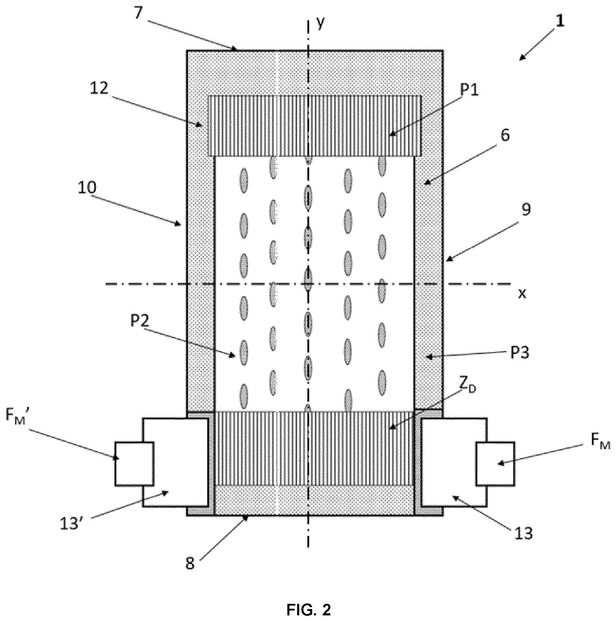

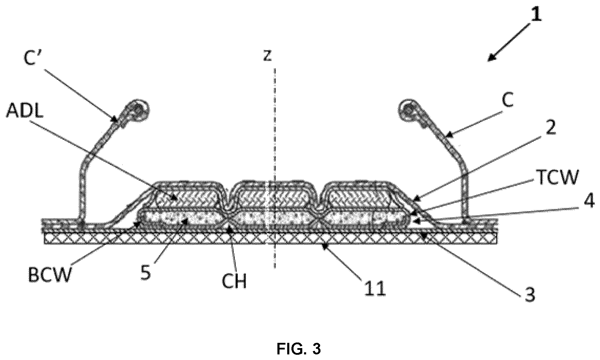

As exemplified in Figs. 1-3, absorbent articles (1) described herein comprise a liquid permeable topsheet (2), a substantially liquid impermeable backsheet (3); and an absorbent core (4) comprising absorbent material (5) therein and being sandwiched between said topsheet (2) and backsheet (3). The topsheet (2), backsheet (3) and absorbent core (4) together generally form a chassis (6) of said article (1), wherein said chassis (6) comprises a perimeter formed by first and second transverse edges (7,8) and first and second longitudinal edges (9,10) connecting the first and second transverse edges (7,8); a longitudinal centerline (y) extending substantially parallel to said first and second longitudinal edges (9,10) and interposed therebetween such to divide said chassis (6) into a first longitudinal half between said longitudinal centerline (y) and said first longitudinal edge (9) and a second longitudinal half between said longitudinal centerline (y) and said second longitudinal edge (10); and a transverse centerline (x) extending substantially parallel to said first and second transverse edges (7,8) and interposed therebetween such to divide said chassis (6) into a first transverse half between said transverse centerline (x) and said first transverse edge (7) and a second transverse half between said transverse centerline (x) and said second transverse edge (8).

Articles herein further comprise an outer cover (11) positioned on a garment-facing side of said backsheet (3) and covering at least 75% of an area defined by said perimeter of said chassis (6) (preferably substantially the entire area, i.e. at least about 100%, of the chassis and/or backsheet generally meaning that the size and/or said area is substantially the same as that of the chassis and/or backsheet); and a fastening system comprising, preferably consisting of, one or more male fasteners (F_{M},F_{M}') and a female fastening material (also referred to as female fastener). The female fastening material comprises a landing zone (12) (generally meaning herein that the female fastening material is in the form of a landing zone) arranged to receive said one or more male fasteners (F_{M},F_{M}') thereon to provide a fastening of said one or more male fasteners (F_{M},F_{M}') to said female fastening material, wherein said female fastening material comprises, preferably consists of, said outer cover (11) generally meaning herein that the female fastening material constitutes (or is) the outer cover such that the outer cover is formed of said female fastening material (i.e. the female fastening material, landing zone and outer cover are all the same component generally having a monolithic structure typically being the same nonwoven material). Advantageously added landing zone material generally used in disposable articles such as diapers may be dispensed with.

In a preferred embodiment, the backsheet (3) comprises, preferably consists of, a breathable, thermoplastic film comprising polyethylene or polypropylene. The breathable, thermoplastic film having a basis weight less than or equal to 15 gsm, preferably from 5 gsm to 14 gsm, even more preferably from 10 gsm to less than 14 gsm, and typically a water vapor transmission rate of at least 500 grams H₂O/24-hour/m². Generally wherein said film has a ratio of the MD load at break to the CD load at break of less than 10, and at least one of a machine direction notched Elmendorf tear strength of at least 5 g or a machine-direction notched trapezoidal tear strength of at least 15 g. Advantageously using lower basis weight film as backsheet enables to significantly reduce cost however normally comes with reduced mechanical performance which is further minimized when using an outer cover and/or joining adhesive patterns as described herein.

The film of the backsheet (3) is preferably selected from a coextruded multilayer film and a monolayer film.

The film of the backsheet (3) may comprise an olefin block copolymer, preferably wherein the olefin block copolymer is a propylene-based polymeric composition and/or an ethylenebased polymeric composition. Preferably wherein the film comprises from about 10% to about 60% of polypropylene.

Preferably, the film of the backsheet (3) comprises from about 30% to about 60% by weight of a filler. Advantageously this permitting to attain the desired breathability and mechanical stability.

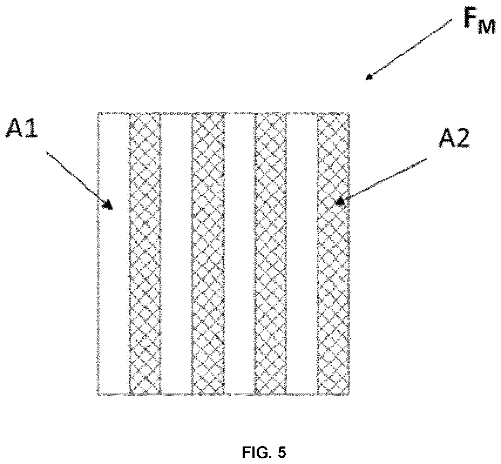

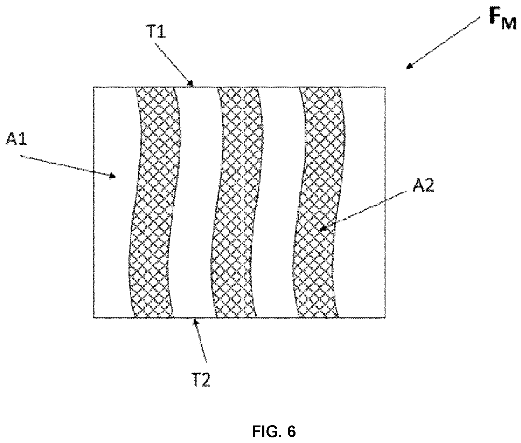

As exemplified in Figs. 1-6, the one or more male fasteners (F_{M},F_{M}') comprise a combination of adhesive and mechanical fastening elements preferably comprising a plurality of hooks. Advantageously having such exposed adhesive has a positive impact on peel strength and dynamic shear stress. Exposed adhesive significantly increases the initial dynamic shear strength which contributes to resistance to shear and allows to reduce the risk of delamination of the outer cover from the backsheet upon application of a shear force which would be higher if only mechanical fasteners are used, yet again adhesive alone does not provide acceptable fastening force when joined to a nonwoven.

The one or more male fasteners (F_{M},F_{M}') comprise one or more first areas of adhesive **A1** and one or more second areas of mechanical fastening elements **A2** (generally in the form of hooks as described in more detail herein) and preferably wherein the total area ratio Σ**A1** / Σ**A2** is greater than or equal to about 0.4, preferably from about 0.4 to about 2.5 (or about 2.0), more preferably between 0.4 and 1.5, more preferably from about 0.45 to about 1.0; even more preferably from about 0.5 to about 0.8, most preferably from about 0.5 to about 0.7, and preferably wherein the outer cover (11) is a nonwoven comprising high-fiber-density areas (HFD) and low-fiber-density areas (LFD), wherein the low-fiber-density areas (LFD) are at least substantially enclosed by high-fiber-density areas (HFD), and wherein the high-fiber-density areas (HFD) are disposed in a repeated pattern (RP) preferably corresponding to embossments of said nonwoven. Although embossed nonwovens are preferred herein, other means of achieving high-fiber-density areas (HFD) and low-fiber-density areas (LFD) are equally encompassed herein, such as by fiber consolidation on meshes having different/varying opening size and distribution, melting of fibers to form dense areas upon solidification, and the like. Advantageously the combination of such tapes/fasteners and outer cover allows for optimal fastening with limited risk of delamination.

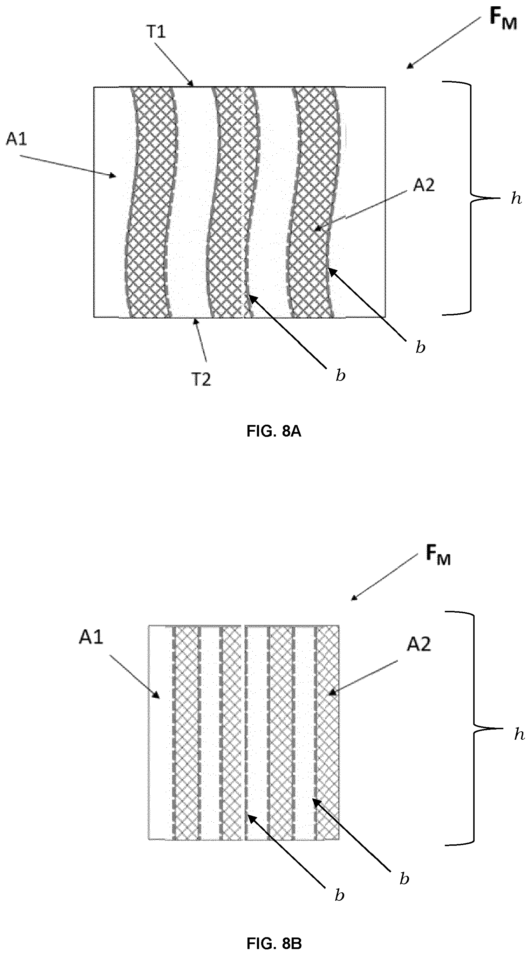

In an embodiment, and as schematically depicted in Figs. 8A-B, the one or more male fasteners (F_{M},F_{M}') comprise a cutting hight *h* (generally extending substantially parallel to the longitudinal centerline (y)) and a plurality of boundary lengths *b* between mechanical fastening elements and adhesive at the border of the areas **A1** and **A2,** and wherein the sum of boundary lengths Σ*b* is preferably greater than 5*h*, even more preferably from 6h to 15*h*, even more preferably from 7*h* to 10*h*, even more preferably from 7*h* to less than *10h.* The boundary lengths *b* generally extending substantially parallel to the cutting height *h*. It has been observed that boundary edges are beneficial in providing initial peel strength, the larger the total distance of such boundaries the stronger the resistance to peel, however when too great there is an increased risk of delamination of the nonwoven and it may thus be beneficial to stay within the described preferred ranges.

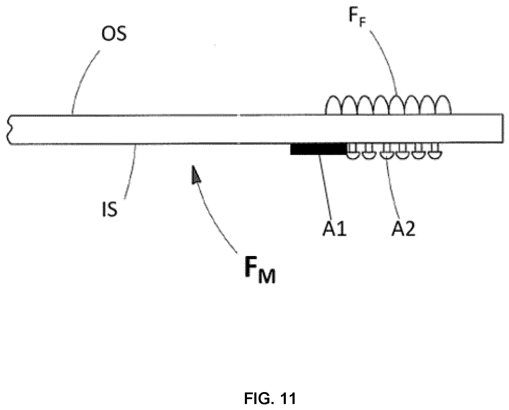

Preferably, the one or more male fasteners (F_{M},F_{M}') herein comprise an inner surface (IS) generally arranged to come into contact with the outer cover (11), and an outer surface (OS) positioned opposite the inner surface (IS). As exemplified in Fig. 11, at least a portion of the inner surface (IS) of the fasteners (F_{M},F_{M}') typically comprises the one or more first areas of adhesive A1 and the one or more second areas of mechanical fastening elements A2.

Generally, articles herein comprise at least two oppositely disposed fasteners (F_{M},F_{M}') comprising a first fastener (F_{M}) and a second fastener (F_{M}'), typically at least one positioned on either side of the longitudinal axis (y) with at least a first fastener (F_{M}) being closer to the first longitudinal edge (9) and further away from the second longitudinal edge (10) compared to the second fastener (F_{M}') generally when viewed in a laid flat and open state as exemplified in Figs.1-2.

Preferably, each of the first fastener (F_{M}) and a second fastener (F_{M}') having at least a portion of an outer surface (OS) thereof comprising a further female fastening material (F_{F}), and wherein the one or more first areas of adhesive A1 and the one or more second areas of mechanical fastening elements A2 of the first fastener (F_{M}) are arranged to come into contact with and/or join to the female fastening material (F_{F}) of the second fastener (F_{M}') or vice versa such to close the article to be worn by a subject. Advantageously this allows for a further securing fit especially for subjects/babies having a smaller waist and/or generally for smaller sizes, yet it may synergistically cooperate with the outer cover by limiting the delamination area when the tapes (or fasteners) are secured one on top of the other and further may aid to apply greater resistance to shear by the stiffening effect created when pressing the one tape over the other.

At least one of, preferably both, first and second fasteners is or are further arranged to come into contact with and/or join to the female fastening material of the outer cover (11) generally in addition to at least one of said first and second fastener being arranged to come into contact with and/or join to the other of the first and second fastener. Advantageously this allows for improved resistance to rotation/twisting during subject's movements whilst , this benefit is further enhanced by both fasters joining to the outer cover (at least portions thereof) and to each other as explained herein above.

In an embodiment, the outer cover (11) is a nonwoven selected from spunbond, carded, spunlace, meltblown, and combinations thereof. When a spunlace nonwoven is selected, said nonwoven preferably comprises a mixture of cellulose and synthetic fibers, preferably wherein the weight ratio of cellulose to synthetic fibers is from about 20:80 to about 75:25, preferably from about 25:75 to about 60:40, more preferably from about 30:70 to about 50:50, even more preferably from about 35:65 to about 45:55. Advantageously this allows for a more natural and sustainable product especially on the largest surface of the article itself. Yet again a sufficient amount of synthetic fibers is beneficial in order to allow for fusing of the fibers in high density areas forming ebossments that are structurally retained by the nonwoven.

In a preferred embodiment, the further female fastening material (F_{F}) is a nonwoven selected from spunbond, carded, spunlace, meltblown, and combinations thereof, and having one or more properties being different from the nonwoven of the outer cover (11). The one or more properties may be selected from the group consisting of basis weight (g/m²), fiber diameter or density, tensile strength in the machine direction, tensile strength in the cross direction, elongation at 10N in machine direction or cross direction, hydrophilicity or hydrophobicity, presence or absence of embossments, and the like.

Preferably the nonwoven of the further female fastening material (F_{F}) has a basis weight that is less (preferably at least 20% less) than the basis weight of the outer cover (11); and/or wherein the nonwoven of the further female fastening material (F_{F}) is hydrophobic and the nonwoven of the outer cover (11) is substantially hydrophilic; and/or wherein the nonwoven of the outer cover (11) is embossed and the nonwoven of the further female fastening material (F_{F}) is substantially free of embossments. Advantageously a different peel force can thus be attained between the fastener-to-fastener connection versus the fastener-to-outer cover connection, with the peel force of the fastener-to-outer cover connection being greater.

When present, the synthetic fibers are preferably hydroentangled with the cellulose fibers in the spunlacing process for forming the spunlace nonwoven. Advantageously this may allow to limit the amount of added binders or other chemical treatments for providing the bonding and fiber network consolidation.

The cellulose fibers may comprise fibers consisting essentially of, preferably consisting of, cotton.

Preferably the outer cover (11) comprises, preferably consists of, a spunbond nonwoven comprising polypropylene fibers. Preferably, the fibers have an average diameter of from about 0.6 dtex and about 2.5 dtex, preferably from about 0.7 dtex to about 2.1 dtex, preferably from about 0.8 dtex to about 2.0 dtex, more preferably from about 0.9 dtex to about 1.8 dtex, for example from about 1 dtex to about 1.5 dtex, or from about 1 dtex to about 1.3 dtex. Advantageously lower average fiber diameter may allow for improved engagement of the hooks, yet the tearing effects normally more associated therewith may be compensated with the added exposed adhesive within the tapes/fasteners hence allowing their use herein which are particularly advantageous when further combined with low basis weight breathable backsheets as described herein above.

Preferably the nonwoven fibers are micro-embossed. Different from embossment of the nonwoven layer (or macro-embossments), micro-embossment of the fibers advantageously allows to reduce the basis weight of the outer cover whilst maintaining anchoring ability of the fasteners thereon. Example of apparatus and processes for making such micro-embossed fibers that may be used herein are described in WO2020/013805 A1 page 9 line 1 to page 11 line 29. Whilst generally high basis weight nonwovens are advantageous in providing anchoring for the fasteners, this adds cost, material usage and hence also waste and negative environmental impact. Using nonwovens having micro-embossed fibers allows to reduce the overall basis weight of the nonwoven yet providing sufficient anchoring surface and have been surprisingly found useful in the application as outer covers.

In a preferred embodiment, the outer cover is a nonwoven and comprises, preferably consists of, fibers that are micro-embossed and the nonwoven is further macro-embossed according to a repeated pattern (RP). Fibers may be polymeric such as comprise or consist of polypropylene and/or polyethylene or may comprise naturally derived or ecological fibers as described in more detail herein. Advantageously combining micro-embossed fibers and a macro-embossed nonwoven comprising such fibers (e.g. by melting fibers of the nonwoven after fiber consolidation in a suitable process e.g. spunlaying. Meltblowing etc. and typically carried out prior to or substantially concurrently with the micro-embossment of the fibers) allows to attain excellent adhesion of the fasteners to the outer cover yet limit the overall basis weight.

The outer cover nonwoven substrate/layer may comprise individual fibers each having a fiber surface and a fiber diameter, wherein a portion of the individual fibers include a micro-embossed pattern, and wherein the micro-embossed pattern includes pattern elements equal to or smaller than the fiber diameter. Preferably, the pattern elements are each 10 microns or less.

The pattern elements may be recessed into and/or extend out of the fiber surface.

The contact angle of the nonwoven substrate may be elevated by 5 or more, preferably by 10 or more, degrees when compared to the same nonwoven substrate without a micro-embossed pattern.

The nonwoven substrate may have a contact angle being greater than 128 degrees.

The micro-embossed pattern may have a pattern roughness, and wherein the pattern roughness is greater than 1.5.

The nonwoven substrate/layer may have a substrate density of less than 0.12 g/cc (g/cm3), preferably less than 0.1 g/cm3, and more preferably less than 0.09 g/cm3. The pattern coverage can be selected as needed for the corresponding application of the substrate as outer cover. In a preferred aspect, the pattern coverage is at least 20%, more preferably at least 40%, more preferably at least 60%, and most preferably at least 70% based on the projected fiber surface area visible from above. Pattern coverage may be measured using digital image analysis. An optical microscope with a 20X objective lens can be used to acquire images, with resolution sufficient to determine which areas of the visible fiber surface contained embossed patterns. Pattern coverage is generally defined as the number of pixels identified as containing pattern in an image divided by the total number of pixels identified as containing fiber in the same image. "Pattern" regions may be identified using an edge detection method to identify only the high contrast areas of the image corresponding to embossed patterns. "Fiber" regions may be identified based on the brightness of the image. Alternatively an image may be converted into black and white and black pixels (vs white pixels) counted.

The spunbond nonwovens for use herein as outer cover (11) preferably comprise more than about 55%wt, preferably more than about 70%wt, even more preferably from about 80%wt to 100%, most preferably from about 90%wt to about 98%wt, of polypropylene fibers by total weight of fibers of the nonwoven. Preferably, the spunbond nonwoven further comprises polyethylene fibers and/or a softening agent in an amount of from about 1%wt to about 40%wt, preferably from about 2%wt to about 30%wt, even more preferably from about 3%wt to about 20%wt, by total weight of the nonwoven. Advantageously this provides a combined mechanical strength and resistance to fiber failure and/or delamination whilst having a degree of softness to the touch.

The basis weight of the outer cover may be from about 5 gsm (g/m²) to about 25 gsm (g/m²), preferably from about 5 gsm (g/m²) to about 20 gsm (g/m²), preferably from 6 gsm to less than 10 gsm, even more preferably from 7 gsm to 9 gsm. Advantageously, embossed nonwovens as described herein above in combination with tapes/fasteners herein allow to select low basis weight nonwovens whilst reducing risk of delamination and hence permitting use as combined cover layer/landing zone with basis weights lower than previously used with the advantages of reduced cost and waste.

In an embodiment, the sum of the low-fiber-density areas (LFD) is greater than about 60%, preferably from about 65% to about 90%, more preferably from about 70% to about 87%, even more preferably from about 75% to about 86%, most preferably from about 75% to about 82%, of a total surface area of the outer cover (11). Advantageously this allows for softness as well as providing bulkiness for hook engagement.

In an embodiment, the sum of the high-fiber-density areas (HFD) is greater than about 10%, preferably from about 11% to about 25%, preferably from about 12% to about 20%, preferably from about 13% to about 18%, more preferably from about 13% to about 25%, even more preferably from about 14% to about 20%, even more preferably from about 15% to about 18%, of a total surface area of the outer cover (11). Without wishing to be bound by theory, the high-fiber-density areas (HFD) particularly interact with the tape/fastener adhesive to provide an effective fastening force yet when hooks are strongly engaged thereto a higher risk of delamination occurs moreover the greater the high-fiber-density areas (HFD) the lower the softness, thus keeping within the above ranges provides an optimal balance between providing surface area for adhesive coupling, minimising risks of delamination, and maximising softness of the outer cover.

Preferably, the fastening tab (herein also referred to as the one or more male fasteners (FM,FM')) has the largest continuous areas of adhesive (i.e. the one or more first areas of adhesive A1) positioned outside of the hook regions (i.e. the one or more second areas of mechanical fastening elements A2), wherein the sum of adhesive areas (i.e. ΣA1) cover and/or overlap more than about 20%, preferably more than about 25%, preferably more than about 30%, preferably from about 32% to about 55%, even more preferably from about 35% to about 50%, of the low-fiber-density areas (LFD), typically of the outer cover (11), in the holding state (i.e. when joined to the landing zone and/or outer cover). Preferably wherein at least one hook-free region (in particular an adhesive region A1) of the fastening tab is arranged between the hook regions. A large adhesive area coverage of the high-loft regions of the nonwoven may be advantageous for greater anchoring strength and resistance to shear. Ensuring an upper limit as described above may however be desirable to limit adhesive peel-off. This embodiment is particularly advantageous when combined with tapes/male fasteners as described herein.

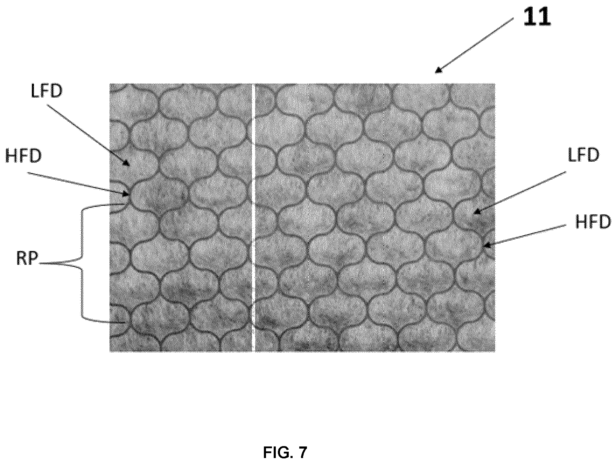

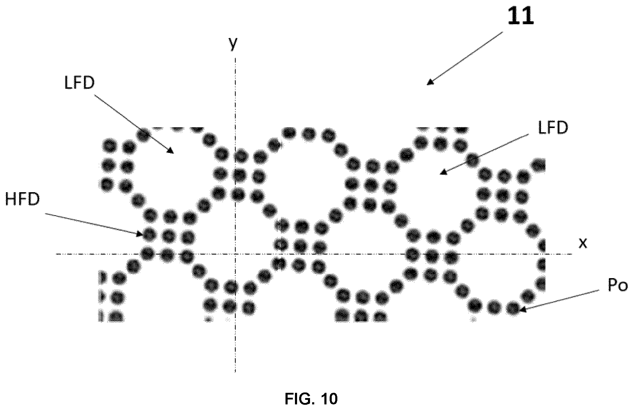

In an embodiment, the repeated pattern (RP) is substantially continuously formed. By continuously formed it is typically intended that a line can be traced along the pattern in absence of interruptions. A number of shapes may be formed as long as the pattern is substantially continuously formed such as for example, diamonds, hexagons, pentagons, octagons, circles and the like and combinations. In an embodiment, the shape comprises octagons or circles as illustrated in Fig. 10. Most preferred are shapes comprising continuous curved lines such as drop-shaped shapes as illustrated in Fig.7.

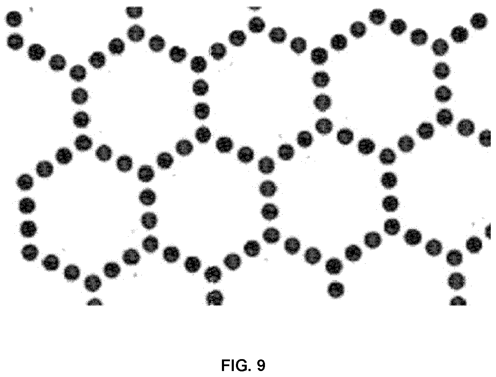

In an embodiment, illustrated in Fig.10, the outer cover (11) comprises a plurality of embossment points (Po) forming high-fiber-density areas (HFD) and one or more low-fiber-density areas (LFD) substantially enclosed by high-fiber-density areas (HFD). The low-fiber-density areas (LFD) may have a first shape selected from hexagons, pentagons, octagons, and circles. Preferably the low-fiber-density areas (LFD) are mutually separated from one another and wherein at least a portion of the said mutually separated low-fiber-density areas (LFD) are separated by a plurality of embossment points (Po) along a substantially transverse axis x and/or substantially longitudinal axis y of the outer cover (generally meaning that said same substantially transverse axis x and/or substantially longitudinal axis y crosses said plurality of embossment points (Po) between two consecutive and/or neighbouring low-fiber-density areas). The said plurality of embossment points (Po) may comprise at least two, preferably at least three, consecutive embossment points (Po) along the same substantially transverse axis x and/or substantially longitudinal axis y. Preferably the said plurality of embossment points (Po) together form one or more polygonal shapes such as square, triangle, star-shape, hexagon, pentagon, octagon, circle, and the like. The said plurality of embossment points (Po) preferably form agglomerates extending along each of the substantially transverse axis x and substantially longitudinal axis y, and generally excludes non-agglomerated point embossment patterns as illustrated for example in the pattern of Fig. 9. Advantageously this allows for localised high density regions of greater surface area between low density regions to help increase the fastening force.

Preferably, the repeated pattern (RP) comprises, preferably consists of, one or more substantially sinusoidal curves. Advantageously this allows for increased contact area that permits higher fastening force along multiple shear axis.

In an embodiment, the repeated pattern (RP) is symmetric about at least the x-axis, y-axis and further axis being about 45° from said x-axis and y-axis. Advantageously this allows for more even fastening force along multiple shear axis.

Preferably, the high-fiber-density areas (HFD) are substantially continuously connected with each other and more preferably such that a plurality of spaced-apart islands of low-fiber-density areas (LFD) are formed therein. Such permits to form highly lofty structures that advantageously increase the surface area of contact to the tapes/fasteners yet provides for a substantially closed/reticulated structure of high density regions for stronger hookengagement.

The one or more male fasteners (F_{M},F_{M}') may be joined and/or comprised on each of at least two oppositely disposed side panels (13,13') that may be elastic. Elastic side panels also referred to as elastic ears in the industry enable to increase the circumferential belly circumference of the article when worn and provide a snug fit. Elastic panels typically comprise two or more nonwoven layers and an elastic film laminated in between said nonwoven layers. Elastic side panels are typically located at the back of the article and joined to the chassis at oppositely disposed locations in the second transverse half of the chassis with at least one on each of the first and second longitudinal halves.

The at least two oppositely disposed side panels (13,13') are preferably joined to the chassis (6) at a garment-facing surface of the backsheet (3) by one or more adhesives and/or mechanical bonding wherein mechanical bonding is selected from pressure, heat, and ultrasonic bonding, and combinations thereof. When joined by adhesive alone, it is preferable that the pattern of adhesive used to join the side panels (13,13') to the backsheet (3) comprises an amount of adhesive per unit area that is greater than the second and/or third bonding pattern (P2, P3).

In an embodiment, the one or more male fasteners (F_{M},F_{M}') comprise one or more first areas of adhesive **A1** and one or more second areas of mechanical fastening elements **A2** and wherein the first area(s) **A1** are mutually separated by second area(s) **A2.** Preferably, the total area ratio Σ**A1** / Σ**A2** is greater than or equal to 0.4, preferably from about 0.4 to about 2.5 (or about 2.0), more preferably between 0.4 and 1.5, more preferably from about 0.45 to about 1.0; even more preferably from about 0.5 to about 0.8, most preferably from about 0.5 to about 0.7. Advantageously this allows for improved peel strength yet limiting risks of delamination. Without wishing to be bound by theory it is believed that adhesive not only contributes to at-start peel resistance but further also contributes to allow adhesion whilst applying a lower force/pressure when joining the tapes/fasteners to the outer cover, at the same time hooks are beneficial for better resistance to shearing yet if too many hooks are present then there is a higher risk of delamination which can be undesirable particularly on repeated use.

Preferably, the one or more second areas of mechanical fastening elements **A2** are alternatingly arranged with the one or more first areas of adhesive **A1** such that said one or more second areas of mechanical fastening elements **A2** are separated by one or more first areas of adhesive **A1.** Advantageously this allows for sequential fortifications resisting to shear.

Preferably, the one or more second areas of mechanical fastening elements **A2** are disposed in a pattern such that each said second area **A2,** when viewed in a planar direction, forms a line-form or sinusoidal shape having a straight and/or curved perimeter.

Preferably the one or more male fasteners (F_{M},F_{M}') each comprise a plurality of second areas of mechanical fastening elements **A2** that are disposed in a pattern such that each said second area **A2,** when viewed in a planar direction, forms a, generally continuous, line-form or sinusoidal shape having a straight and/or curved perimeter and wherein neighbouring said second areas **A2** are separated by one or more areas of adhesive **A1.** Said second areas **A2** each generally having a length and a width, said length being substantially parallel to a cutting hight h of the one or more male fasteners (F_{M},F_{M}') and said width being perpendicular thereto; wherein said second areas **A2** comprise an aspect ratio (width/length) of less than 0.13, preferably from 0.05 to 0.12, more preferably from 0.06 to 0.11. Advantageously, thinner straight or curved stripes of hooks allow for good adhesion with the application of a lower force making it more easy and user-friendly in-use.

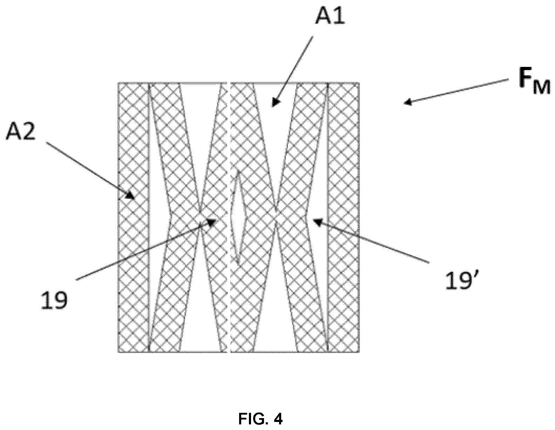

Less preferred and preferably to be avoided are fasteners, such as illustrated in Fig. 4, that comprise one or more second areas of mechanical fastening elements **A2** form a mesh structure (19) with one or more first areas of adhesive **A1** forming one or more adhesive patches (19') substantially encircled by one or more second areas of mechanical fastening elements **A2.** Such fasteners add cost and further make it more challenging to include the desired higher amount of exposed adhesive described herein.

Preferably, the one or more first areas of adhesive **A1,** when viewed in a planar direction, forms a line-form or sinusoidal shape having a straight and/or curved perimeter, and wherein each of said first areas of adhesive **A1** continuously extends from a first terminal position (T1) to a second terminal position (T2). Typically wherein the first terminal position (T1) and second terminal position (T2) are adjacent to respective upper and lower transverse edges of the male fastener (F_{M},F_{M}'). An advantageous peel strength can be hence achieved on nonwovens by maximising adhesive continuity along the length and/or width of the tape(s)/fastener(s).

In a preferred embodiment, the one or more male fasteners (F_{M},F_{M}') comprise a first adhesive and a second adhesive, wherein the first and second adhesives are different (i.e. have different compositional properties). The first adhesive is comprised in the one or more first areas of adhesive **A1** and the second adhesive is comprised in the one or more second areas **A2** such to join the mechanical fastening elements to said male fasteners (F_{M},F_{M}'). Preferably a portion of the second adhesive remains exposed (i.e. at least a portion of the second adhesive in the one or more second areas **A2** is free of mechanical fastening elements such to expose said portion of second adhesive typically for coming into contact with one or more fibers of the outer cover 11). Advantageously, not only are the advantages mentioned above relating to the adhesive/hook (or mechanical fasteners) bonding to the outer cover still attained but further the exposed adhesive contributes to improving sheer resistance yet reduce the amount of hooks covering the second areas A2 such to limit delamination of the fibers of the outer cover when peeling and/or releasing the male fasteners from the outer cover and achieve an added releasable holding force between hooks.

Preferably, the first adhesive comprises a first amount of tackifier and the second adhesive comprises a second amount of tackifier, and the first amount of tackifier is greater than the second amount of tackifier; preferably wherein the first amount of tackifier is at least 5% greater, preferably from 10% to 200% greater, more preferably from 15% to 150% greater, even more preferably from 20% to 100% greater, than the second amount of tackifier. Advantageously this allows to increase the speed at which a bond is formed when the first adhesive is brought into contact with the outer cover even with very slight pressure which allows to provide for an initial resistance to sheer while the mechanical fasteners/hooks are engaged.

The adhesive A1 may comprise a pressure-sensitive adhesive having a modulus at 50% elongation of 68,650 to 117,680 Pa and a modulus at 200% elongation of 147,100 to 245,170 Pa. Advantageously, a practically satisfactory adhesive strength of the pressure-sensitive adhesive tape to the back-sheet of a disposable diaper is obtained even when non-woven fabric is used as the back-sheet of the disposable diaper. The moduli of the pressure-sensitive adhesive preferably has a modulus at 50% elongation of 78,450 to 117,680 Pa and a modulus at 200% elongation of 166,710 to 225,550 Pa.

In an embodiment, the pressure-sensitive adhesives for use herein may be selected from the group consisting of rubber-based pressure-sensitive adhesives, acrylic-based pressure-sensitive adhesives, silicone-based pressure-sensitive adhesive, and combinations thereof.

It may be preferred to use particularly those pressure-sensitive adhesives which contain as a major component (main polymer) styrene-based block copolymers selected from styreneisoprene-styrene block copolymers (SIS), styrene-butadiene-styrene block copolymers (SBS), and styrene-ethylene/butylene-styrene block copolymers (SEBS).

When the above-described styrene-based block copolymers are used as a main component of the pressure-sensitive adhesive, the pressure-sensitive adhesive used preferably is a pressure-sensitive adhesive comprising 100 parts by weight of styrene block copolymer, 5 to 30 parts by weight, more preferably 10 to 25 parts by weight of a softener which is liquid at a normal temperature (23° C), 110 to 200 parts by weight of a tackifier which is solid at a normal temperature (23° C).

The backsheet (3) and the outer cover (11) are preferably joined together by one or more adhesives, wherein the landing zone (12) of the outer cover (11) is joined to the backsheet (3) by a first joining pattern (P1) and the area of the outer cover (11) outboard of the landing zone (12) is joined to the backsheet (3) by a second joining pattern (P2), wherein the first joining pattern (P1) comprises a greater amount of adhesive(s) per unit area than the second joining pattern (P2). By ensuring a higher amount of adhesive per unit area (e.g. higher basis weight in gsm or g/m² or greater surface area coverage) is applied greater mechanical integrity of the nonwoven material is provided that is better able to resist particularly to shear forces generally associated with tape fasteners. Yet again in other areas of the backsheet having a lower amount of adhesive per unit area permits to save on cost and material waste as well as providing improved overall softness to the touch.

Preferably, the amount per unit area as used herein is the basis weight in g/m² of adhesive. The first pattern (P1) thus preferably comprises a higher basis weight of adhesive compared to the second pattern (P2) and that is preferably greater than 30%, preferably at least 50%, more preferably at least 70%, even more preferably from 80% to 400%, most preferably from 95% to 300%, greater than the basis weight of adhesive in the second joining pattern (P2). Typically, "joining pattern" is intended herein as the entire area within which the referenced pattern is present (e.g. the second joining pattern P2 encompasses within its meaning the backsheet area that is subjected to such pattern i.e. the sum of the areas of the pattern itself as well as adjacent areas thereto where no other pattern is present, as shown in the figures by for example the sum of the white and shaded areas within a generally rectangular frame).

The amount per unit area may be the surface area coverage of adhesive relative to a total surface area of one or more regions of the backsheet. This may for example be inspected under UV-light and image analysis to then determine the respective surface area coverage. Advantageously this may allow for in-line automated camera inspection of the products and hence be integrated into quality control processes automatically carried out in production.

In an embodiment, the amount of adhesive(s) per unit area comprised in the first joining pattern (P1) is at least 30%, preferably at least 50%, more preferably at least 70%, even more preferably from 80% to 400%, most preferably from 95% to 300%, greater than the amount of adhesive(s) per unit area comprised in the second joining pattern (P2).

In an embodiment, the backsheet (3) and the outer cover (11) are joined together by a third bonding pattern (P3) located substantially outboard of the first and second bonding pattern (P1, P2) and along at least a portion of the perimeter of the chassis (6), wherein the third joining pattern (P3) comprises an amount of adhesive(s) per unit area that is greater than or equal to that of the first joining pattern (P1). Advantageously this allows for sealing of the cover layer at the edges in a secure manner that limits inadvertent delamination during production and/or in use.

The first bonding pattern (P1) may comprise one or more first stripes or swirls of 22dhesivee, and wherein the second bonding pattern (P2) may comprise a plurality of second stripes or swirls of adhesive, typically wherein the first stripe(s) have an aspect ratio that is greater than that of the second stripes, the aspect ratio being defined as a ratio of a longest dimension and a smallest dimension of said stripes. Advantageously this allows for air channels or gaps to be formed between joining areas which are of greater size/volume hence improving softness to the touch though whilst this is beneficial over the majority of the surface area of the outer cover (coming to skin contact with a caregiver who places the article onto a subject) it is undesirable for the landing zone as higher risks of delamination is promoted.

In an embodiment, the first and/or second bonding pattern (P1, P2) comprises a plurality of bonding points having a circular or elliptical shape, and preferably having an aspect ratio of from about 0.1 to about 4.5, preferably from about 0.5 to about 4.0, more preferably from about 0.8 to about 3.5, even more preferably from about 0.9 to about 3.0, even more preferably from about 1 to about 2.5; and more preferably wherein the distance between neighbouring bonding points of the second bonding pattern (P2) is greater than the distance between neighbouring bonding points of the first bonding pattern (P1). Advantageously this allows for increased non-joined contact area around the bonding points promoting loftiness and softness.

Preferably, the landing zone (12) comprises a main landing zone positioned at a front portion of the chassis (6) that is closer to the first transverse edge (7) and further from the second transverse edge (8) and typically within the first transversal half of the chassis (6); and preferably a disposal-landing-zone (Z_{D}) positioned at a back portion of the chassis (6) that is closer to the second transverse edge (8) and further from the first transverse edge (7) and typically in the second transversal half of the chassis (6), said disposal-landing-zone (Z_{D}) adapted for receiving the one or more male fasteners (F_{M}) thereon once the absorbent article is rolled-up and/or folded for disposal thereof. Advantageously this allows for optimal closure of the male fasteners onto the appropriate region of the outer cover once the soiled article e.g. diaper is rolled up to contain the exudates therein and then fastened to remain in its substantially rolled-up state ready for easy disposal in a clean and non-messy manner.

Outer cover (11) layers for use herein may be selected from nonwovens comprising plant-based fibers wherein said plant-based fibers comprise, preferably consist of, harvested fibers other than wood pulp, such that said topsheet (2) has a bio-based content of from about 10% to about 100%, preferably from about 15% to about 100%, even more preferably from about 20% to about 100%, using ASTM D6866-10, method B, and wherein said nonwoven further comprises synthetic fibers generally in an amount to provide added structural integrity such as from 25% to 85%, generally from 30% to 65%, by weight of said nonwoven.

Backsheets for use herein may be breathable and/or comprise a film, preferably polyethylene (PE) based or bio-PE based.

The absorbent core (4) may comprise a core wrap enclosing the absorbent material therein. The core wrap may comprise a top core wrap layer (TCW) and a bottom core wrap layer (BCW) that are joined at a periphery thereof to contain absorbent material therein and avoid leakage towards a skin of a wearer. The top core wrap layer (TCW) and a bottom core wrap layer (BCW) may be different layers or the same core wrap layer that is folded to form top and bottom layers.

Preferably, the absorbent core (4) comprises one or more attachment zones wherein the top layer (TCW) of the core wrap is adhered to the lower layer (BCW) of the core wrap such that one or more channels (CH) substantially free of absorbent material are formed, preferably inboard of a perimeter of said core (4) so that said channels (CH) do not extend to or reach said perimeter. The channels (CH) may extend from 10% to 85%, preferably from 15% to 75%, of a length of the core (4) extending in a direction substantially parallel to the longitudinal axis (y). Advantageously this allows to reduce the list of leakage as well as to provide improved cup formation when worn.

In a preferred embodiment, channels (CH) herein are longitudinally extending and cross both the transverse centerline (x) at a first crossing point and the longitudinal centerline (y) at a second crossing point, wherein the first and second crossing points are different and preferably wherein the second crossing point is positioned closer to the second transverse edge (8) than the first crossing point, and preferably wherein the second transverse edge (8) is positioned proximal to the back region of the article.

Articles herein may further comprise an acquisition distribution layer (ADL) positioned between the topsheet and the absorbent core, preferably between the topsheet and a top core wrap layer. The acquisition distribution layer may have a basis weight of from 15 gsm to 55 gsm, preferably from 18 gsm to 50 gsm, even more preferably from 19 gsm to 45 gsm. Preferably the acquisition distribution layer is selected from a carded air-through bonded nonwoven, a carded thermobonded calendered nonwoven, a spunbond nonwoven, and combinations thereof.

The articles herein may further comprise a wetness indicator that is viewable from a garment-facing side of the backsheet (3) and outer cover (11), wherein said indicator is applied to said garment-facing side of the backsheet (3). Typically wherein the wetness indicator is positioned between a garment-facing side of the core wrap and a body-facing surface of the backsheet (3), preferably positioned between a garment-facing side of the bottom/lower core wrap (BCW) and a body/skin-facing surface of the backsheet (3).

Absorbent articles herein may comprise a pair of barrier cuffs extending along the first and second longitudinal edges (C, C') and arranged to provide lateral barriers preventing exudate leakage. Typically, the barrier cuffs comprising a hydrophobic nonwoven and one or more elastics at an apex position thereof such to form standing gathers.

In an embodiment, the absorbent material comprises, preferably consists of, superabsorbent particles and/or cellulose fibers.

The absorbent cores herein may comprise at least 60%wt of superabsorbent particles, in particular at least 70%wt, preferably at least 80%wt, preferably at least 90%wt, by total weight of the core.

### EXAMPLES

The following materials are tested by a panel of at least 10, preferably 100, panelists. Each panelist is provided with one sample product for each of the combinations of Tapes A/B with outer covers NW1/NW2/NW3 (as described below). All diaper samples being identical apart from a mono-variable change of either tape (according to tapes A or B) or nonwoven outer cover (according to nonwovens NW1, NW2 or NW3). The samples are free of separate frontal tape and rather the panelists are instructed to join the tapes directly to the outer cover. The panelists are asked to test each of the samples to assess the tape engagement onto respective outer cover. Panelists are asked to provide a rating as follows:

**Table 1 - ratings and descriptor**

| **Descriptor** | **Rating** |
|---|---|
| Poor: the fastening force is not adequate as it takes several attempts to get to a good closing and when peeling the tape from the outer cover the outer cover fibers are pulled away with clear delamination being observed; | - |
| Neutral: neither poor nor good | **0** |
| Good: the fastening force is good as the tape stays very securely in position after first application and when peeling the tape from the outer cover little to no outer cover fibers are pulled away with no or minor delamination being observed | + |

Tapes/Fasteners:
**TAPE A** - Lohmann Koester (CP 1/M 46 NG FHL4 H6). Such tapes have the following characteristics: ΣA1(exposed adhesive area) = 155 mm²; ΣA2 (Hook area) = 310 mm²; Area ratio Σ**A1**/Σ**A2 = 0.50**
**TAPE B** - 3M (Mechanical Laminate PH White NWFT). Such tapes have the following characteristics: ΣA1(exposed adhesive area) = 113 mm²; ΣA2 (Hook area) = 286 mm²; Area ratio **ΣA1/ΣA2 = 0.39**

Outer covers:
**NW 1** - Spunbond nonwoven 100% polypropylene fibers having a basis weight of 16 gsm and embossed with G8 pattern and manufactured and sold by PFN (Humboldt Industrial Park, 69 Green Mountain Rd, Hazle Township, PA 18202, United States). With pattern as exemplified in Fig. 7. The high-fiber-density areas (HFD) is 14% (of total surface area).
**NW 2 -** Spunbond nonwoven 100% polypropylene fibers having a basis weight of 16 gsm and embossed with pillow pattern and manufactured and sold by Union Industries SpA (Via II Giugno, 80, 13866 Masserano BI, Italy). With pattern as exemplified in Fig. 10. The high-fiber-density areas (HFD) is 18% (of total surface area).
**NW 3** - Spunbond nonwoven 100% polypropylene fibers having a basis weight of 16 gsm and embossed with hexa-dot pattern and manufactured and sold by Fitesa Italy Srl (Via Bologna 7, Trezzano Rosa (MI), Italy). With pattern as exemplified in Fig. 9. The high-fiber-density areas (HFD) is 11% (of total surface area).

**Table 2 - Results of the panel test**

| With **NW 1** as outer cover: | | |
|---|---|---|
| | **TAPE A (%)** | **TAPE B (%)** |
| **-** | **0%** | **20%** |
| **0** | **50%** | **50%** |
| **+** | **50%** | **30%** |

### With NW 2 as outer cover:

| | **TAPE A (%)** | **TAPE B (%)** |
|---|---|---|
| **-** | **10%** | **10%** |
| **0** | **0%** | **70%** |
| **+** | **90%** | **20%** |

### With NW 3 as outer cover:

| | **TAPE A (%)** | **TAPE B (%)** |
|---|---|---|
| **-** | **10%** | **40%** |
| **0** | **70%** | **50%** |
| **+** | **20%** | **10%** |

The results show that Tape A scores best on all outer covers NW1-NW3. Yet NW1 allows for Tape B to close the gap in performance. Similarly, NW2 provides for excellent performance particularly in combination with Tape A.

It is supposed that the present invention is not restricted to any form of realization described previously and that some modifications can be added to the presented embodiments without reappraisal of the appended claims.

## Claims

1. A disposable absorbent article (1) comprising:
a liquid permeable topsheet (2);
a substantially liquid impermeable backsheet (3); and
an absorbent core (4) comprising absorbent material (5) therein and being sandwiched between said topsheet (2) and backsheet (3);
the topsheet (2), backsheet (3) and absorbent core (4) together forming a chassis (6) of said article (1), wherein said chassis (6) comprises a perimeter formed by first and second transverse edges (7,8) and first and second longitudinal edges (9,10) connecting the first and second transverse edges (7,8); a longitudinal centerline (y) extending substantially parallel to said first and second longitudinal edges (9,10) and interposed therebetween such to divide said chassis (6) into a first longitudinal half between said longitudinal centerline (y) and said first longitudinal edge (9) and a second longitudinal half between said longitudinal centerline (y) and said second longitudinal edge (10); and a transverse centerline (x) extending substantially parallel to said first and second transverse edges (7,8) and
interposed therebetween such to divide said chassis (6) into a first transverse half between said transverse centerline (x) and said first transverse edge (7) and a second transverse half between said transverse centerline (x) and said second transverse edge (8);
wherein said article further comprises an outer cover (11) positioned on a garment-facing side of said backsheet (3) and covering at least 75% of an area defined by said perimeter of said chassis (6); and a fastening system comprising one or more male fasteners (F_{M},F_{M}') and a female fastening material;
and wherein the female fastening material comprises a landing zone (12) arranged to receive said one or more male fasteners (F_{M},F_{M}') thereon to provide a fastening of said one or more male fasteners (F_{M},F_{M}') to said female fastening material, wherein said female fastening material comprises, preferably consists of, said outer cover (11), and wherein the one or more male fasteners (F_{M},F_{M}') comprise a combination of adhesive and mechanical fastening elements;
**characterised in that** the one or more male fasteners (F_{M},F_{M}') comprise one or more first areas of adhesive **A1** and one or more second areas of mechanical fastening elements **A2** and wherein the total area ratio Σ**A1 /** Σ**A2** is greater than or equal to 0.4 and **in that** the outer cover (11) is a nonwoven, preferably an embossed nonwoven, comprising high-fiber-density areas (HFD) and low-fiber-density areas (LFD), wherein the low-fiber-density areas (LFD) are at least substantially enclosed by high-fiber-density areas (HFD), and wherein the high-fiber-density areas (HFD) are disposed in a repeated pattern (RP), preferably corresponding to embossments of said nonwoven.

2. An absorbent article (1) according to Claim 1 wherein the first area(s) **A1** are mutually separated by second area(s) **A2.**

3. An absorbent article (1) according to any of the preceding Claims comprising at least two oppositely disposed fasteners (F_{M},F_{M}') comprising a first fastener (F_{M}) and a second fastener (F_{M}'), and wherein each of the first fastener (F_{M}) and a second fastener (F_{M}') having at least a portion of an outer surface (OS) thereof comprising a further female fastening material (F_{F}), and wherein the one or more first areas of adhesive A1 and the one or more second areas of mechanical fastening elements A2 of the first fastener (F_{M}) are arranged to come into contact with and/or join to the female fastening material (F_{F}) of the second fastener (F_{M}') or vice versa such to close the article to be worn by a subject.

4. An absorbent article (1) according to any of the preceding Claims wherein the total area ratio Σ**A1** / Σ**A2** is from about 0.45 to about 2.0, preferably from about 0.5 to about 1.0, even more preferably from about 0.5 to about 0.8.

5. An absorbent article (1) according to any of the preceding Claims wherein the sum of the low-fiber-density areas (LFD) is from about 75% to about 87%, of a total surface area of the outer cover (11); and wherein the sum of the high-fiber-density areas (HFD) is from about 13% to about 20%, preferably from about 14% to about 18%, of a total surface area of the outer cover (11).

6. An absorbent article (1) according to any of the preceding Claims wherein the repeated pattern (RP) is substantially continuously formed; and/or wherein the repeated pattern (RP) comprises, preferably consists of, one or more substantially sinusoidal curves; and/or wherein the repeated pattern (RP) is symmetric about at least the x-axis, y-axis and further axis being about 45° from said x-axis and y-axis.

7. An absorbent article (1) according to any of the preceding Claims, wherein the high-fiber-density areas (HFD) are substantially continuously connected with each other.

8. An absorbent article (1) according to any of the preceding Claims wherein the one or more second areas of mechanical fastening elements **A2** are alternatingly arranged with the one or more first areas of adhesive **A1** such that said one or more second areas of mechanical fastening elements **A2** are separated by one or more first areas of adhesive **A1.**

9. An absorbent article (1) according to any of the preceding Claims wherein the one or more second areas of mechanical fastening elements **A2** are disposed in a pattern such that each said second area **A2,** when viewed in a planar direction, forms a line-form or sinusoidal shape having a straight and/or curved perimeter.

10. An absorbent article (1) according to any of the preceding Claims wherein the one or more first areas of adhesive **A1,** when viewed in a planar direction, forms a line-form or sinusoidal shape having a straight and/or curved perimeter, and wherein each of said first areas of adhesive **A1** continuously extends from a first terminal position (T1) to a second terminal position (T2).

11. An absorbent article (1) according to any of the preceding Claims wherein the backsheet (3) and the outer cover (11) are preferably joined together by one or more adhesives, wherein the landing zone (12) of the outer cover (11) is joined to the backsheet (3) by a first joining pattern (P1) and the area of the outer cover (11) outboard of the landing zone (12) is joined to the backsheet (3) by a second joining pattern (P2), wherein the first joining pattern (P1) comprises a greater amount of adhesive(s) per unit area than the second joining pattern (P2).

12. An absorbent article (1) according to Claim 11 wherein the backsheet (3) and the outer cover (11) are joined together by a third bonding pattern (P3) located substantially outboard of the first and second bonding pattern (P1, P2) and along at least a portion of the perimeter of the chassis (6), wherein the third joining pattern (P3) comprises an amount of adhesive(s) per unit area that is greater than or equal to that of the first joining pattern (P1).

13. An absorbent article (1) according to any one of Claims 11 to 12 wherein the first bonding pattern (P1) comprises one or more first stripes or swirls of adhesive, and wherein the second bonding pattern (P2) comprises a plurality of second stripes or swirls of adhesive, wherein the first stripe(s) have an aspect ratio that is greater than that of the second stripes, the aspect ratio being defined as a ratio of a longest dimension and a smallest dimension of said stripes; and/or wherein the first and/or second bonding pattern (P1, P2) comprises a plurality of bonding points having a circular or elliptical shape, and preferably having an aspect ratio of from 0.1 to 4.5, and more preferably wherein the distance between neighbouring bonding points of the second bonding pattern (P2) is greater than the distance between neighbouring bonding points of the first bonding pattern (P1).

14. An absorbent article (1) according to any of the preceding Claims wherein the landing zone (12) comprises a main landing zone positioned at a front portion of the chassis (6) that is closer to the first transverse edge (7) and further from the second transverse edge (8); and preferably a disposal-landing-zone (Z_{D}) positioned at a back portion of the chassis (6) that is closer to the second transverse edge (8) and further from the first transverse edge (7) said disposal-landing-zone (Z_{D}) adapted for receiving the one or more male fasteners (F_{M}) thereon once the absorbent article is rolled-up and/or folded for disposal thereof.

15. An absorbent article (1) according to any of the preceding Claims wherein the one or more male fasteners (F_{M},F_{M}') comprise a cutting hight *h* and a plurality of boundary lengths b between mechanical fastening elements and adhesive at the border of the areas **A1** and **A2,** and wherein the sum of boundary lengths Σ*b* is preferably greater than 5*h*, even more preferably from 6*h* to 15*h,* even more preferably from 7*h* to 10*h*, even more preferably from 7h to less than *10h.*

16. An absorbent article (1) according to any of the preceding Claims wherein the backsheet (3) comprises a breathable thermoplastic film comprising polyethylene or polypropylene and said film having a basis weight less than or equal to 15 gsm and a water vapor transmission rate of at least 500 grams H₂O/24-hour/m²; and/or wherein the outer cover (11) is a nonwoven having a basis weight of from about 5 g/m² to about 20 g/m², and wherein said nonwoven comprises fibers having an average diameter of from about 0.6 dtex to about 2.5 dtex; and/or wherein the backsheet (3) and the outer cover (11) are joined together by a plurality of adhesive bonding patterns wherein said patterns are different in at least one of shape and/or basis weight.

17. An absorbent article (1) according to any of the preceding Claims wherein the one or more male fasteners (FM,FM') comprise a first adhesive and a second adhesive, wherein the first and second adhesives are different, preferably wherein the first areas of adhesive **A1** comprise the first adhesive and the second areas of mechanical fastening elements **A2** comprise the second adhesive joining said mechanical fastening elements to said male fasteners (FM,FM') and arranged such that a portion of the second adhesive remains exposed and capable of coming into direct contact with one or more fibers of the outer cover (11).

18. An absorbent article (1) according to any of the preceding Claims wherein the outer cover (11) is a nonwoven comprising fibers that are micro-embossed, and preferably wherein the nonwoven is further macro-embossed with a pattern corresponding to the repeated pattern (RP).

## Patentansprüche

1. Absorbierender Einwegartikel (1), umfassend:
eine flüssigkeitsdurchlässige Oberschicht (2);
eine im Wesentlichen flüssigkeitsundurchlässige Unterschicht (3); und
einen absorbierenden Kern (4), umfassend absorbierendes Material (5) darin, der zwischen der Oberschicht (2) und der Unterschicht (3) angeordnet ist;
wobei die Oberschicht (2), Unterschicht (3) und der absorbierende Kern (4) zusammen einen Unterbau (6) des Artikels (1) bilden, wobei der Unterbau (6) einen Umfang umfasst, der durch erste und zweite Querkanten (7,8) und erste und zweite Längskanten (9, 10), die die ersten und zweiten Querkanten (7,8) verbinden, gebildet wird; eine Längsmittellinie (y), die sich im Wesentlichen parallel zu den ersten und zweiten Längskante (9, 10) erstreckt und derart dazwischen eingefügt ist, dass der Unterbau (6) in eine erste Längshälfte zwischen der Längsmittellinie (y) und der ersten Längskante (9) und eine zweite Längshälfte zwischen der Längsmittellinie (y) und der zweiten Längskante (10) geteilt ist; und eine Quermittellinie (x), die sich im Wesentlichen parallel zu den ersten und zweiten Querkanten (7, 8) erstreckt und derart dazwischen eingefügt ist, dass der Unterbau (6) in eine erste Querhälfte zwischen der Quermittellinie (x) und der ersten Querkante (7) und eine zweite Querhälfte zwischen der Quermittellinie (x) und der zweiten Querkante (8) geteilt ist;
wobei der Artikel ferner eine äußere Abdeckung (11) umfasst, die auf einer kleidungszugewandten Seite der Unterschicht (3) positioniert ist und mindestens 75 % einer durch den Umfang des Unterbaus (6) definierten Fläche abdeckt; und ein Befestigungssystem, umfassend einen oder mehrere männliche Befestigungselemente (F_{M},F_{M}') und ein weibliches Befestigungsmaterial;
und wobei das weibliche Befestigungsmaterial einen Aufnahmebereich (12) umfasst, der angeordnet ist, um eine oder mehrere männliche Befestigungselemente (F_{M},F_{M}') darauf aufzunehmen, um eine Befestigung von einen oder mehreren männlichen Befestigungselementen (F_{M},F_{M}') am weiblichen Befestigungsmaterial bereitzustellen, wobei das weibliche Befestigungsmaterial die äußere Abdeckung (11) umfasst, vorzugsweise daraus besteht, und wobei die einen oder mehreren männlichen Befestigungselemente (F_{M},F_{M}') eine Kombination aus Klebstoff und mechanischen Befestigungselementen umfassen;
**dadurch gekennzeichnet, dass** die einen oder mehreren männlichen Befestigungen (F_{M},F_{M}') einen oder mehrere erste Bereiche aus Klebstoff **A1** und einen oder mehrere zweite Bereiche aus mechanischen Befestigungselementen **A2** umfassen und wobei das Gesamtflächenverhältnis **ΣA1** / **ΣA2** größer als oder gleich 0,4 ist und **dadurch, dass** die äußere Abdeckung (11) ein Vliesstoff ist, vorzugsweise ein geprägter Vliesstoff, umfassend Bereiche hoher Faserdichte (HFD) und Bereiche niedriger Faserdichte (LFD), wobei die Bereiche niedriger Faserdichte (LFD) mindestens im Wesentlichen von Bereichen hoher Faserdichte (HFD) umschlossen sind, und wobei die Bereiche hoher Faserdichte (HFD) in einem wiederholten Muster (RP) angeordnet sind, vorzugsweise entsprechend Prägungen des Vliesstoffs.

2. Absorbierender Artikel (1) nach Anspruch 1, wobei der/die erste(n) Bereich(e) **A1** durch zweite(n) Bereich(e) **A2** voneinander getrennt sind.

3. Absorbierender Artikel (1) nach einem der vorstehenden Ansprüche, umfassend mindestens zwei gegenüberliegend angeordnete Befestigungselemente (F_{M},F_{M}'), umfassend ein erstes Befestigungselement (F_{M}) und ein zweites Befestigungselement (F_{M}'), und wobei jedes des ersten Befestigungselements (F_{M}) und eines zweiten Befestigungselements (F_{M}') mindestens einen Abschnitt einer Außenoberfläche (OS) davon aufweist, der ein weiteres weibliches Befestigungsmaterial (F_{F}) umfasst, und wobei der eine oder die mehreren ersten Bereiche aus Klebstoff A1 und der eine oder die mehreren zweiten Bereiche aus mechanischen Befestigungselementen A2 des ersten Befestigungselements (F_{M}) angeordnet sind, um mit dem weiblichen Befestigungsmaterial (F_{F}) des zweiten Befestigungselements (F_{M}') in Kontakt zu kommen und/oder sich damit zu verbinden oder umgekehrt, um den Artikel zu schließen, der von einem Subjekt getragen werden soll.

4. Absorbierender Artikel (1) nach einem der vorstehenden Ansprüche, wobei das Gesamtflächenverhältnis **ΣA1 / ΣA2** von etwa 0,45 bis etwa 2,0, vorzugsweise von etwa 0,5 bis etwa 1,0, noch mehr bevorzugt von etwa 0,5 bis etwa 0,8 beträgt.

5. Absorbierender Artikel (1) nach einem der vorstehenden Ansprüche, wobei die Summe der Bereiche mit geringer Faserdichte (LFD) etwa 75 % bis etwa 87 % einer gesamten Oberfläche der äußeren Abdeckung (11) beträgt; und wobei die Summe der Bereiche mit hoher Faserdichte (HFD) etwa 13 % bis etwa 20 %, vorzugsweise etwa 14 % bis etwa 18 %, einer Gesamtoberfläche der äußeren Abdeckung (11) beträgt.

6. Absorbierender Artikel (1) nach einem der vorstehenden Ansprüche, wobei das wiederholte Muster (RP) im Wesentlichen kontinuierlich gebildet ist; und/oder wobei das wiederholte Muster (RP) eine oder mehrere im Wesentlichen sinusförmige Kurven umfasst, vorzugsweise daraus besteht; und/oder wobei das wiederholte Muster (RP) symmetrisch bezüglich mindestens der x-Achse, y-Achse und einer weiteren Achse ist, die etwa 45° von der x-Achse und y-Achse entfernt ist.

7. Absorbierender Artikel (1) nach einem der vorstehenden Ansprüche, wobei die Bereiche mit hoher Faserdichte (HFD) im Wesentlichen kontinuierlich miteinander verbunden sind.

8. Absorbierender Artikel (1) nach einem der vorstehenden Ansprüche, wobei der eine oder die mehreren zweiten Bereiche von mechanischen Befestigungselementen **A2** abwechselnd mit dem einen oder den mehreren ersten Bereichen aus Klebstoff **A1** angeordnet sind, sodass der eine oder die mehreren zweiten Bereiche aus mechanischen Befestigungselementen **A2** durch einen oder mehrere erste Bereiche aus Klebstoff **A1** getrennt sind.

9. Absorbierender Artikel (1) nach einem der vorstehenden Ansprüche, wobei der eine oder die mehreren zweiten Bereiche von mechanischen Befestigungselementen **A2** in einem Muster derart angeordnet sind, dass jeder zweite Bereich **A2,** wenn in einer planaren Richtung betrachtet, eine linienförmige oder sinusförmige Form mit einem geraden und/oder gekrümmten Umfang bildet.

10. Absorbierender Artikel (1) nach einem der vorstehenden Ansprüche, wobei der eine oder die mehreren ersten Bereiche aus Klebstoff **A1,** wenn in einer planaren Richtung betrachtet, eine linienförmige oder sinusförmige Form mit einem geraden und/oder gekrümmten Umfang bilden, und wobei jeder der genannten ersten Bereiche aus Klebstoff **A1** sich kontinuierlich von einer ersten Endposition (T1) zu einer zweiten Endposition (T2) erstreckt.

11. Absorbierender Artikel (1) nach einem der vorstehenden Ansprüche, wobei die Unterschicht (3) und die äußere Abdeckung (11) vorzugsweise durch einen oder mehrere Klebstoffe miteinander verbunden sind, wobei der Aufnahmebereich (12) der äußeren Abdeckung (11) mit der Unterschicht (3) durch ein erstes Verbindungsmuster (P1) verbunden ist und der Bereich der äußeren Abdeckung (11) außerhalb der Landezone (12) mit der Unterschicht (3) durch ein zweites Verbindungsmuster (P2) verbunden ist, wobei das erste Verbindungsmuster (P1) eine größere Menge an Klebstoff(en) pro Flächeneinheit umfasst als das zweite Verbindungsmuster (P2).

12. Absorbierender Artikel (1) nach Anspruch 11, wobei die Unterschicht (3) und die äußere Abdeckung (11) durch ein drittes Verbindungsmuster (P3) miteinander verbunden sind, das im Wesentlichen außerhalb des ersten und zweiten Verbindungsmusters (P1, P2) und entlang mindestens eines Teils des Umfangs des Unterbaus (6) angeordnet ist, wobei das dritte Verbindungsmuster (P3) eine Menge an Klebstoff(en) pro Flächeneinheit umfasst, die größer oder gleich derjenigen des ersten Verbindungsmusters (P1) ist.

13. Absorbierender Artikel (1) nach einem der Ansprüche 11 bis 12, wobei das erste Verbindungsmuster (P1) einen oder mehrere erste Streifen oder Kreise aus Klebstoff umfasst und wobei das zweite Verbindungsmuster (P2) eine Vielzahl von zweiten Streifen oder Kreisen aus Klebstoff umfasst, wobei der erste Streifen/die ersten Streifen ein Seitenverhältnis aufweist/aufweisen, das größer ist als das der zweiten Streifen, wobei das Seitenverhältnis als ein Verhältnis einer längsten Abmessung und einer kleinsten Abmessung der Streifen definiert ist; und/oder wobei das erste und/oder zweite Verbindungsmuster (P1, P2) eine Vielzahl von Verbindungspunkten umfasst, die eine kreisförmige oder elliptische Form aufweisen, und vorzugsweise ein Seitenverhältnis von 0,1 bis 4,5 aufweisen, und mehr bevorzugt wobei der Abstand zwischen benachbarten Verbindungspunkten des zweiten Verbindungsmusters (P2) größer ist als der Abstand zwischen benachbarten Verbindungspunkten des ersten Verbindungsmusters (P1).

14. Absorbierender Artikel (1) nach einem der vorstehenden Ansprüche, wobei der Aufnahmebereich (12) einen Hauptaufnahmebereich umfasst, der an einem vorderen Abschnitt des Unterbaus (6) positioniert ist, der näher zu der ersten Querkante (7) und weiter von der zweiten Querkante (8) entfernt ist; und vorzugsweise einen Entsorgungsaufnahmebereich (Z_{D}), der an einem hinteren Abschnitt des Unterbaus (6) positioniert ist, der näher zu der zweiten Querkante (8) und weiter von der ersten Querkante (7) entfernt ist, wobei der Entsorgungsaufnahmebereich (Z_{D}) dafür ausgelegt ist, die einen oder mehreren männlichen Befestigungselemente (F_{M}) darauf aufzunehmen, sobald der absorbierende Artikel für dessen Entsorgung aufgerollt und/oder gefaltet ist.

15. Absorbierender Artikel (1) nach einem der vorstehenden Ansprüche, wobei der eine oder die mehreren männlichen Befestigungselemente (F_{M},F_{M}') eine Schnitthöhe h und eine Vielzahl von Grenzlängen b zwischen mechanischen Befestigungselementen und Klebstoff an der Grenze der Bereiche **A1** und **A2,** umfassen, und wobei die Summe der Grenzlängen Σ*b* vorzugsweise größer als 5h, noch mehr bevorzugt von 6*h* bis 15*h*, noch mehr bevorzugt von 7*h* bis 10*h,* noch mehr bevorzugt von 7*h* bis weniger als 10*h* ist.

16. Absorbierender Artikel (1) nach einem der vorstehenden Ansprüche, wobei die Unterschicht (3) eine atmungsaktive thermoplastische Folie umfasst, die Polyethylen oder Polypropylen umfasst, und wobei die Folie ein Flächengewicht von weniger als oder gleich 15 g/m² und eine Wasserdampfdurchlässigkeitsrate von mindestens 500 Gramm H₂O/24-Stunden/m² aufweist; und/oder wobei die äußere Abdeckung (11) ein Vliesstoff mit einem Flächengewicht von etwa 5 g/m² bis etwa 20 g/m² ist, und wobei der Vliesstoff Fasern mit einem durchschnittlichen Durchmesser von etwa 0,6 dtex bis etwa 2,5 dtex umfasst; und/oder wobei die Unterschicht (3) und die äußere Abdeckung (11) durch eine Vielzahl von Klebemustern miteinander verbunden sind, wobei sich die Muster in mindestens einem von Form und/oder Flächengewicht unterscheiden.

17. Absorbierender Artikel (1) nach einem der vorstehenden Ansprüche, wobei die einen oder mehreren männlichen Befestigungselemente (FM,FM') einen ersten Klebstoff und einen zweiten Klebstoff umfassen, wobei sich der erste und der zweite Klebstoff unterscheiden, vorzugsweise wobei die ersten Bereiche aus Klebstoff **A1** den ersten Klebstoff umfassen und die zweiten Bereiche aus mechanischen Befestigungselementen **A2** den zweiten Klebstoff umfassen, der die mechanischen Befestigungselemente mit den männlichen Befestigungselementen (FM,FM') verbindet und derart angeordnet ist, dass ein Teil des zweiten Klebstoffs freiliegend bleibt und in der Lage ist, in direkten Kontakt mit einer oder mehreren Fasern der äußeren Abdeckung (11) zu kommen.

18. Absorbierender Artikel (1) nach einem der vorstehenden Ansprüche, wobei die äußere Abdeckung (11) ein Vliesstoff ist, der Fasern umfasst, die mikrogeprägt sind, und vorzugsweise wobei der Vliesstoff ferner mit einem Muster makrogeprägt ist, das dem wiederholten Muster (RP) entspricht.

## Revendications

1. Article absorbant jetable (1) comprenant :
une feuille de dessus perméable aux liquides (2) ;
une feuille de fond (3) sensiblement imperméable aux liquides ; et
un noyau absorbant (4) comprenant un matériau absorbant (5) à l'intérieur de celui-ci et étant placé en sandwich entre ladite feuille de dessus (2) et ladite feuille de fond (3) ;
la feuille de dessus (2), la feuille de fond (3) et le noyau absorbant (4) formant ensemble un châssis (6) dudit article (1), dans lequel ledit châssis (6) comprend un périmètre formé par des premier et second bords transversaux (7,8) et des premier et second bords longitudinaux (9,10) reliant les premier et second bords transversaux (7,8) ; une ligne médiane longitudinale (y) s'étendant sensiblement parallèlement auxdits premier et second bords longitudinaux (9,10) et interposée entre eux de façon à diviser ledit châssis (6) en une première moitié longitudinale entre ladite ligne médiane longitudinale (y) et ledit premier bord longitudinal (9) et une seconde moitié longitudinale entre ladite ligne médiane longitudinale (y) et ledit second bord longitudinal (10) ; et une ligne médiane transversale (x) s'étendant sensiblement parallèlement auxdits premier et second bords transversaux (7,8) et interposée entre eux de façon à diviser ledit châssis (6) en une première moitié transversale entre ladite ligne médiane transversale (x) et ledit premier bord transversal (7) et une seconde moitié transversale entre ladite ligne médiane transversale (x) et ledit second bord transversal (8) ;
dans lequel ledit article comprend en outre une couverture externe (11) positionnée sur un côté faisant face vers le vêtement de ladite feuille de fond (3) et couvrant au moins 75 % d'une zone définie par ledit périmètre dudit châssis (6) ; et un système de fixation comprenant un ou plusieurs éléments de fixation mâles (F_{M},F_{M}') et un matériau de fixation femelle ;
et dans lequel le matériau de fixation femelle comprend une zone de réception (12) agencée pour recevoir ledit un ou plusieurs éléments de fixation mâles (F_{M},F_{M}') sur celle-ci pour fournir une fixation dudit un ou plusieurs éléments de fixation mâles (F_{M},F_{M}') audit matériau de fixation femelle, dans lequel ledit matériau de fixation femelle comprend, de préférence est constitué de, ladite couverture externe (11), et dans lequel le ou les éléments de fixation mâles (F_{M},F_{M}') comprennent une combinaison d'éléments adhésifs et de fixation mécanique ;
**caractérisé en ce que** le ou les éléments de fixation mâles (F_{M},F_{M}') comprennent une ou plusieurs premières zones d'adhésif **A1** et une ou plusieurs secondes zones d'éléments de fixation mécaniques **A2** et dans lequel le rapport d'aire total **ΣA1 / ΣA2** est supérieur ou égal à 0,4 et **en ce que** la couverture externe (11) est un non-tissé, de préférence un non-tissé gaufré, comprenant des zones à haute densité de fibres (HFD) et des zones à faible densité de fibres (LFD), dans lequel les zones à faible densité de fibres (LFD) sont au moins sensiblement entourées de zones à haute densité de fibres (HFD), et dans lequel les zones à haute densité de fibres (HFD) sont disposées selon un motif répété (RP), correspondant de préférence à des gaufrages dudit non-tissé.

2. Article absorbant (1) selon la revendication 1, dans lequel la ou les premières zones **A1** sont mutuellement séparées par la ou les secondes zones **A2.**

3. Article absorbant (1) selon l'une quelconque des revendications précédentes, comprenant au moins deux éléments de fixation (Fₘ,Fₘ') disposés de manière opposée, comprenant un premier élément de fixation (F_{M}) et un second élément de fixation (F_{M}'), et dans lequel chacun parmi le premier élément de fixation (F_{M}) et un second élément de fixation (F_{M}') ayant au moins une partie d'une surface externe (OS) de celui-ci, comprenant un matériau de fixation femelle supplémentaire (F_{F}), et dans lequel la ou les premières zones d'adhésif A1 et la ou les secondes zones d'éléments de fixation mécaniques A2 du premier élément de fixation (F_{M}) sont agencées pour entrer en contact et/ou se joindre au matériau de fixation femelle (F_{F}) du second élément de fixation (F_{M}'), ou vice versa, de manière à fermer l'article destiné à être porté par un sujet.

4. Article absorbant (1) selon l'une quelconque des revendications précédentes, dans lequel le rapport de surface totale **ΣA1** / **ΣA2** est d'environ 0,45 à environ 2,0, de préférence d'environ 0,5 à environ 1,0, encore plus préférablement d'environ 0,5 à environ 0,8.

5. Article absorbant (1) selon l'une quelconque des revendications précédentes, dans lequel la somme des zones à faible densité de fibres (LFD) est d'environ 75 % à environ 87 % d'une surface totale de la couverture externe (11) ; et dans lequel la somme des zones à haute densité de fibres (HFD) est d'environ 13 % à environ 20 %, de préférence d'environ 14 % à environ 18 %, d'une surface totale de la couverture externe (11).

6. Article absorbant (1) selon l'une quelconque des revendications précédentes, dans lequel le motif répété (RP) est formé de façon sensiblement continue ; et/ou dans lequel le motif répété (RP) comprend, de préférence est constitué de, une ou plusieurs courbes sensiblement sinusoïdales ; et/ou dans lequel le motif répété (RP) est symétrique par rapport à au moins l'axe x, l'axe y et un autre axe étant à environ 45° desdits axe x et axe y.

7. Article absorbant (1) selon l'une quelconque des revendications précédentes, dans lequel les zones à haute densité de fibres (HFD) sont reliées de manière sensiblement continue les unes aux autres.

8. Article absorbant (1) selon l'une quelconque des revendications précédentes, dans lequel la ou les secondes zones d'éléments de fixation mécaniques **A2** sont agencées alternativement avec la ou les premières zones d'adhésif **A1** de telle sorte que ladite une ou les secondes zones d'éléments de fixation mécaniques **A2** sont séparées par une ou plusieurs premières zones d'adhésif **A1.**

9. Article absorbant (1) selon l'une quelconque des revendications précédentes, dans lequel la ou les secondes zones d'éléments de fixation mécaniques **A2** sont disposées dans un motif de telle sorte que chaque dite seconde zone **A2,** lorsqu'elle est vue dans une direction plane, forme une forme de ligne ou une forme sinusoïdale ayant un périmètre droit et/ou incurvé.

10. Article absorbant (1) selon l'une quelconque des revendications précédentes, dans lequel la ou les premières zones d'adhésif **A1,** lorsqu'elles sont vues dans une direction plane, forment une forme de ligne ou une forme sinusoïdale ayant un périmètre droit et/ou incurvé, et dans lequel chacune desdites premières zones d'adhésif **A1** s'étend de manière continue d'une première position terminale (T1) à une seconde position terminale (T2).

11. Article absorbant (1) selon l'une quelconque des revendications précédentes, dans lequel la feuille de fond (3) et la couverture externe (11) sont de préférence reliées ensemble par un ou plusieurs adhésifs, dans lequel la zone de réception (12) de la couverture externe (11) est jointe à la feuille de fond (3) par un premier motif de jonction (P1) et la zone de la couverture externe (11) à l'extérieur de la zone de réception (12) est jointe à la feuille de fond (3) par un second motif de jonction (P2), dans lequel le premier motif de jonction (P1) comprend une plus grande quantité d'adhésif(s) par unité de surface que le second motif de jonction (P2).

12. Article absorbant (1) selon la revendication 11, dans lequel la feuille de fond (3) et la couverture externe (11) sont reliées ensemble par un troisième motif de liaison (P3) situé sensiblement à l'extérieur du premier et du deuxième motif de liaison (P1, P2) et le long d'au moins une partie du périmètre du châssis (6), dans lequel le troisième motif de liaison (P3) comprend une quantité d'adhésif(s) par unité de surface qui est supérieure ou égale à celle du premier motif de liaison (P1).

13. Article absorbant (1) selon l'une quelconque des revendications 11 à 12, dans lequel le premier motif de liaison (P1) comprend une ou plusieurs premières bandes ou tourbillons d'adhésif, et dans lequel le second motif de liaison (P2) comprend une pluralité de secondes bandes ou tourbillons d'adhésif, dans lequel la ou les premières bandes ont un rapport d'aspect qui est supérieur à celui des secondes bandes, le rapport d'aspect étant défini comme un rapport entre une dimension la plus longue et une plus petite dimension desdites bandes ; et/ou dans lequel le premier et/ou le second motif de liaison (P1, P2) comprend une pluralité de points de liaison ayant une forme circulaire ou elliptique, et ayant de préférence un rapport d'aspect de 0,1 à 4,5, et plus préférablement dans lequel la distance entre des points de liaison voisins du second motif de liaison (P2) est supérieure à la distance entre des points de liaison voisins du premier motif de liaison (P1).

14. Article absorbant (1) selon l'une quelconque des revendications précédentes, dans lequel la zone de réception (12) comprend une zone de réception principale positionnée au niveau d'une partie avant du châssis (6) qui est plus proche du premier bord transversal (7) et plus éloignée du second bord transversal (8) ; et de préférence une zone de réception d'élimination (Zo) positionnée au niveau d'une partie arrière du châssis (6) qui est plus proche du second bord transversal (8) et plus éloignée du premier bord transversal (7), ladite zone de réception d'élimination (Z_{D}) étant destinée à recevoir le ou les éléments de fixation mâles (F_{M}) sur celle-ci une fois que l'article absorbant est enroulé et/ou plié pour être jeté.

15. Article absorbant (1) selon l'une quelconque des revendications précédentes, dans lequel le ou les éléments de fixation mâles (F_{M},F_{M}') comprennent une hauteur de coupe h et une pluralité de longueurs limites b entre des éléments de fixation mécaniques et de l'adhésif au niveau du bord des zones **A1** et **A2,** et dans lequel la somme des longueurs limites Σ*b* est de préférence supérieure à 5*h*, encore plus préférablement de 6*h* à 15*h*, encore plus préférablement de 7*h* à 10*h,* encore plus préférablement de 7*h* à moins de *10h.*

16. Article absorbant (1) selon l'une quelconque des revendications précédentes, dans lequel la feuille de fond (3) comprend un film thermoplastique respirant comprenant du polyéthylène ou du polypropylène et ledit film ayant une masse surfacique inférieure ou égale à 15 grammes par mètre carré et un taux de transmission de vapeur d'eau d'au moins 500 grammes de H₂O/24 heures/m² ; et/ou dans lequel la couverture externe (11) est un non-tissé ayant une masse surfacique d'environ 5 g/m² à environ 20 g/m², et dans lequel ledit non-tissé comprend des fibres ayant un diamètre moyen allant d'environ 0,6 dtex à environ 2,5 dtex ; et/ou dans lequel la feuille de fond (3) et la couverture externe (11) sont reliées ensemble par une pluralité de motifs de liaison adhésive, dans lequel lesdits motifs sont différents dans au moins l'une parmi une forme et/ou une masse surfacique.

17. Article absorbant (1) selon l'une quelconque des revendications précédentes, dans lequel le ou les éléments de fixation mâles (FM,FM') comprennent un premier adhésif et un second adhésif, dans lequel les premier et second adhésifs sont différents, de préférence dans lequel les premières zones d'adhésif **A1** comprennent le premier adhésif et les secondes zones d'éléments de fixation mécaniques **A2** comprennent le second adhésif joignant lesdits éléments de fixation mécaniques auxdits éléments de fixation mâles (FM,FM') et agencés de telle sorte qu'une partie du second adhésif reste exposée et capable d'entrer en contact direct avec une ou plusieurs fibres de la couverture externe (11).

18. Article absorbant (1) selon l'une quelconque des revendications précédentes, dans lequel la couverture externe (11) est un non-tissé comprenant des fibres qui sont micro-gaufrées, et de préférence dans lequel le non-tissé est en outre macro-gaufré avec un motif correspondant au motif répété (RP).
